# EUROPEAN PATENT SPECIFICATION

(11) **EP 0 585 912 B1**
(45) Date of publication and mention of the grant of the patent: **16.07.1997**
(21) Application number: 93114023.0
(22) Date of filing: 02.09.1993
(51) Int. Cl.: G01N 33/558, G01N 33/543, G01N 33/58

(54) **Chromatographic sandwich test for detection of specific antibody and device therefor**
Chromatographischer Sandwichtest zur Bestimmung von spezifischen Antikörpern und Vorrichtung hierfür
Essai de sandwich chromatografique pour la détection des anticorps spécifiques et dispositif a cet effet

(30) Priority: 04.09.1992 US 940598
(43) Date of publication of application: 09.03.1994
(73) Proprietor: Becton Dickinson and Company, Franklin Lakes, New Jersey 07417-1880 (US)
(72) Inventor: Lovell, Stephen J., Towson, Maryland 21204 (US)
(74) Representative: von Kreisler, Alek, Dipl.-Chem.

(56) References cited:
- EP-A- 0 462 376
- WO-A-91/12528
- US-A- 4 228 237

## Description

### BACKGROUND OF INVENTION

Immunoassays are now well known tools of the diagnostic industry. Such assays take advantage of the specificity of the antigen antibody reaction to detect the presence of the analyte in the test sample. This provides a simple method for measuring the analyte.

When the target analyte is an antibody, the assay ordinarily uses an anti-species antibody coated on the detector, such as anti-IgG or anti-IgM. This type of detector is difficult to incorporate into a single step assay, as it would tend to bind both specific and non-specific antibody, if the non-specific antibody is in excess in the sample. Such is generally the case with serum (See for example EP-A-0 462 376).

Also, if the target antigen is used, in a "sandwich" assay, to improve the sensitivity, the above problems are complicated by the fact that such antigens are often expensive and/or difficult to obtain in the quantities needed for the assay.

Thus, there exists a need for alternate technologies to utilize such a single step assay.

### SUMMARY OF INVENTION

This invention presents a simple and rapid single step, indirect assay for the detection of specific antibodies in a sample. Specifically, the test utilizes a three zone chromatographic device wherein the three zones are: a biotinylated antigen zone; a detector zone; and a capture zone.

The biotinylated antigen zone contains antigen or antigen analog conjugated with biotin. This zone is preferably upstream of the detector zone, which contains anti-biotin antibody bound to detector particulates. This zone is, in turn, upstream of the capture zone which contains antigen or antigen analog bound to the chromatographic matrix. As an alternative, the detector zone can be placed upstream of the biotinylated antigen zone.

In the assay, the sample is applied upstream of the biotinylated antigen zone. The sample migrates downstream and any specific antibody will bind to the biotinylated antigen, which in turn will bind the anti-biotin/detector particulates in the detector zone, or, in the alternative embodiment, this order is reversed. These complexes will then migrate to the capture zone where the multivalent specific antibody will bind to the bound antigen. Any complexes, thus bound, can be detected by the detector signal.

### BRIEF DESCRIPTION OF THE FIGURES

FIGURE 1 depicts a chromatographic device of this invention showing the chromatographic matrix (1), biotinylated antigen zone (2), detector zone (3), and capture zone (4).

FIGURE 2 depicts the complex formed in the capture zone showing the antibiotin-detector (1), biotinylated antigen (2), antigen bound to the chromatographiic matrix (3) and specific antibody (4).

### DETAILED DESCRIPTION OF INVENTION

The biotinylated antigen zone comprises antigen conjugated with biotin. The conjugation can be by any method known in the art, the only criterion being the requirement that such method not affect the structure of either the antigen or the biotin to the extent that either would not bind to antibody (i.e. the specific antibody for the antigen or the anti-biotin antibody for the biotin).

The preferred method of conjugation of antigen to biotin is by reaction of amino groups on the antigen with N-hydroxysuccinimidobiotin. If antigen does not contain at least one amino group, it may be possible to chemically treat the antigen to introduce amino groups. Alternative chemical pathways are also available, for example, periodate oxidation or carbodiimide treatment of antigen followed by reaction with biotin hydrazide.

The use of biotinylated antigen obviates the need for conjugation of antigen to detector particulates.

In a preferred embodiment the detector zone comprises particles of detector coated with antibiotin-antibody, which is easily and relatively inexpensively obtained. For a true one step assay, the detector should provide a visible readout, although other detectors (e.g. fluorescent, UV) can also be used, the only criterion being the ability to attach anti-biotin antibody to its surface.

The detector is a particulate material. One useful particulate is a sac which includes a dye or other colored substance as a readout. The sac which is used may be any one of a wide variety of sacs, including but not limited to intact erythrocytes, erythrocyte ghosts, liposomes (single walled, sometimes called vesicles or multilamellar), polymer microcapsules (for example, those made by coascervation, or interfacial polymerization), etc. Also useful are colored particles (e.g., latex or other polymeric particles) and precipitated or insoluble metals, nonmetals and alloys.

The primary criterion for selection of the detector particulates are the ability to bind the anti-biotin antibody and the visibility of the readout under test conditions.

The signal-generating substance can be any substance which exhibits a detectable signal under test conditions, but is preferably a visible colored dye or pigment. Other dyes, such as UV or fluorescent, can also be used, but will complicate detection by necessitating the use of specialized equipment for detection. Most preferably the detector is a colored latex particle.

The anti-biotin antibody is attached to the surface of the detector, by means which are well known in the art, in a geometric orientation which renders it accessible to reaction with biotin in the specific antibody/biotinylated antigen/anti-biotin complex to form a secondary complex. The preferred method of attachment of anti-biotin antibody to colored latex is by activation of carboxy latex with EDC (1-ethyl-3-(3-dimethyl-aminopropyl)-carbodiimide) followed by incubation of the activated latex with antibody.
Attachment of antibody to the latex is, thus, through covalent coupling of lysine residues of the antibody to carboxyl groups on the surface of the latex.

This secondary complex then migrates through the chromatographic matrix to the capture zone which contains antigen immobilized on the matrix by means which are well known in the art, preferably, by direct application of a small volume of antigen containing solution by micropipettor or by spraying (e.g. CAMAG Linomat 1V applicator): in both cases application is followed by a drying step (45°C for 30 min).

The multivalent specific antibody also binds to the bound antigen in this zone to form a labeled biotinylated antigen/ antibiotin/specific antibody bound antigen "sandwich," which can then be detected.

The assay is highly selective for specific antibody, since it relies on the formation of an antigen/antibody complex which is similarly highly specific.

It is further noted that while the preferred embodiment of the device of this invention has the detector zone downstream of the biotinylated antibody zone, these two can be reversed without ill effect, as only secondary complexes containing specific antibody will bind to the capture zone and generate a detectable signal.

The chromatographic matrix can be any of the various matrices known in the art including silica, polyacrylamide alumina, and, preferably, nitrocellulose. Further, while the assay is preferably performed in a thin layer chromatograph device (TLC) format, it can also be performed in any other compatible chromatographic formats such as column chromatography.

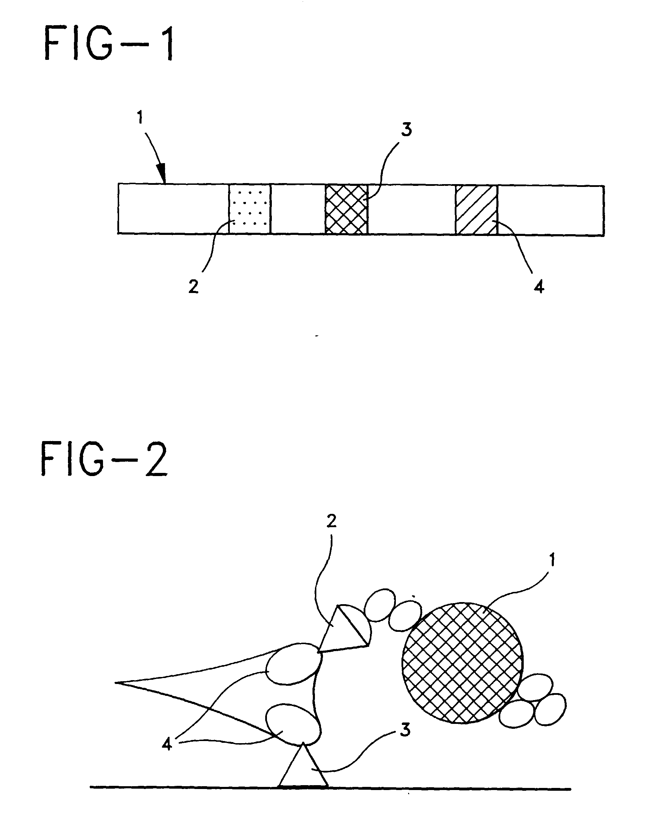

In the preferred embodiment, the TLC device is arranged as shown in FIG. 1. Briefly, the sample is introduced to the chromatographic matrix (1) upstream of the biotinylated antigen zone (2), which is upstream of the detector zone (3). After migrating through these two zones, the sample continues on to the capture zone (4) where any secondary complexes are bound and detected.

### EXAMPLES

The following examples are illustrative of certain preferred embodiments of this invention, but are not intended to be illustrative of all embodiments.

### Example 1 - Conjugation of hemocyanin with biotin

To illustrate conjugation of hemocyanin to biotin, the following procedure was run:

N-hydroxysuccinimidobiotin (4 mg/ml in dimethyl sulfoxide) was added dropwise (with stirring) to antigen (keyhole limpet hemocyanin mg/ml 1 in 0.1 M NaHCO₃, pH 8.2). Specifically, the solution of N-hydroxysuccinimidobiotin was added to the antigen solution in the ratio 1:20 (v/v), and the mixture was allowed to stir for 4 hours at room temperature. After 4 hours, the reaction was quenched by the addition of 0.1 volume of glycine ethyl ester, pH 7.5. The resulting mixture was then dialyzed exhaustively against 0.1 M NaHCO₃, pH 8.2, to removed unconjugated biotin, and the product was retained.

### Example 2 - conjugation of anti-biotin to latex

To illustrate attachment of antibiotin antibody to latex, the following procedure was run:
1. Blue colored latex particles (nominally 0.5µm diameter), 10% (W/V) in 1 ml water were pelleted by centrifugation (12,000g for 10 min) and resuspended in 1.25 ml of 4 mM aqueous KH₂PO₄.
2. The latex was then activated by the addition of 2 ml of 2% EDC (1-ethyl-3-(3-dimethylaminopropyl)-carbodiimide). The pH of the mixture was adjusted to 5.0 by the addition of 2 N HCl and the mixture was then stirred for 90 minutes at room temperature.
3. After the 90 minutes, the mixture was pelleted by centrifugation (12,000g/10min.) and resuspended in 10 ml of 0.17 M aqueous NaCl. The latex was again centrifuged and the pellet resuspended with 1.25 ml of 0.2 M aqueous sodium borate, pH 8.5.
4. The suspension of activated latex in borate was then mixed with 1.25 ml of rabbit anti-biotin antibody (2 mg/ml in 0.2 M sodium borate, pH 8.5), and stirred for 90 min at room temperature.
5. The reaction was then immediately quenched by the addition of 0.01 ml of 1 M ethanolamine followed by a 30 minute incubation at room temperature. After the 30 minute, 0.1 ml of 5% BSA in 0.2 M aqueous sodium borate, pH 8.5, was added and the incubation was continued for a further 30 minutes.
6. The mixture was then pelleted by centrifugation, as above, and resuspended in 10 ml of Resuspension Solution (0.2% BSA, 0.1 M glycine, 0.17 M NaCl, 0.2% sodium azide, pH 8.2). The latex was then pelleted again and resuspended thoroughly in 15 ml of Resuspension Solution. The final suspension of anti-biotin coated latex was retained and stored refrigerated.

### Example 3 - Detection of Rabbit antihemocyanin antibody, biotinylated antigen upstream of detector.

A thin layer chromatographic device was prepared as follows:
1. Nitrocellulose (12µm pore size) was cut into strips approximately 6 cm long and 0.8 cm wide. Keyhole limpet hemocyanin (KLH) (3µl at 3.90 mg/ml) was applied as a dot by micropipettor approximately 3.5 cm from the upstream end of each strip to form the capture zone.
2. Blue latex detector coated with anti-biotin antibody (Example 2) was diluted 4-fold with Diluting Solution (10% BSA, 10% lactose, 0.2% Triton™ X-100 in 10 mM sodium phosphate, 15 mM sodium chloride, pH 7.7), and applied by micropipettor as a zone 2.5 cm from the upstream end of each strip (6 µl of diluted latex per strip).
3. Biotinylated keyhole limpet hemocyanin (Example 1) was diluted to a final concentration of 0.2 mg/ml with Diluting Solution. The diluted solution of biotinylated KLH was applied as a zone by micropipettor 1.5 cm from the upstream end of each nitrocelullose strip (6 µl per strip).
4. The strips were dried for 30 min at 45°C.

The dried strips were then used in an assay. Briefly, the test serum (containing 1% Triton™ X-100) was applied to the upstream end of each strip. Depending on the serum, the results varied as follows:
a. Application of rabbit anti-hemocyanin serum (Research Plus, Inc.) resulted in an intense blue color in the region where unmodified KLH was spotted (i.e., 3.5 cm from the upstream end).
b. Application of non-immune rabbit serum did not result in any color being trapped at the KLH capture zone.
c. Addition of hemocyanin to the rabbit anti-hemocyanin serum (final concentration of KLH was 0.6 mg/ml) prior to application of the serum to the strip resulted in a markedly reduced signal (compared to that observed for unblocked immune serum).

Thus, the assay clearly exhibits the presence of anti-hemocyanin antibody.

### Example 4 - Detection of Rabbit antihemocyanin antibody, biotinylated antigen downstream of detector.

The procedure of Example 3 was repeated except that the KLH (capture zone) was spotted on the strip 3 cm from the upstream end, biotinylated KLH 2cm from the upstream end, and latex antibody 1 cm from the upstream end.

In the assay, the results were essentially equivalent to Example 3, i.e., unblocked serum containing antibody showed an intense blue color in the capture zone, non-immune serum showed no color in the capture zone, and blocked serum containing antibody showed a marked decrease in color in the capture zone.

## Claims

1. A device for performing single step immunoassays for specific antibodies to an antigen comprising a chromatographic matrix containing a biotinylated antigen zone, a detector zone and a capture zone downstream of said biotinylated antigen and detector zone, wherein the biotinylated antigen zone contains the antigen bound to biotin, said detector zone comprises detector particulates coated with an anti-biotin antibody, and said capture zone contains the antigen or an antigen analog bound to the chromatographic matrix.

2. The device of Claim 1 wherein the chromatographic matrix is selected from the group consisting of silica, nylon, glass fiber, polyacrylamide, alumina, and nitrocellulose.

3. The device of Claim 1 wherein the detector particulates produce a visible readout.

4. The device of Claim 1 wherein the detector is selected from the group consisting of precipitated or insoluble metals, precipitated or insoluble nonmetals, sacs containing a dye or colored substance and precipitated or insoluble alloys.

5. A method for determining the presence of specific antibody to an antigen in a sample comprising:
(i) applying the sample to a chromatographic matrix of a device, containing a biotinylated antigen zone, a detector zone and a capture zone, wherein said sample is applied upstream of the three zones;
(ii) allowing said sample to migrate firstly through said biotinylated antigen zone, which contains antigen conjugated to biotin, and said detector zone, which contains detector particulates coated with an anti-biotin antibody, such that antigen-antibody complexes are formed, and subsequently through said capture zone, which contains the antigen or an antigen analog bound to said chromatographic matrix, such that the antigen-antibody complexes formed will bind to said antigen or antigen analog; and
(iii) observing the capture zone for the presence of particulates, wherein the presence of particulates indicates the presence of the specific antibody.

6. The method of Claim 5 wherein the chromatographic matrix is selected from the group consisting of silica, nylon, glass fiber, polyacrylamide, alumina, and nitrocellulose.

7. The method of Claim 5 wherein the detector particulates produce a visible readout.

8. The method of Claim 5, wherein the detector is selected from the group consisting of precipitated or insoluble metals, precipitated or insoluble nonmetals, sacs containing a dye or colored substance and precipitated or insoluble alloys.

9. The method of Claim 5 wherein said sample migrates through said biotinylated antigen zone before migrating through the detector zone.

10. The method of Claim 5 wherein said sample migrates through said biotinylated antigen zone after said detector zone.

## Patentansprüche

1. Vorrichtung zur Durchführung von einstufigen Immunoassays für spezifische Antikörper gegen ein Antigen, umfassend eine chromatographische Matrix, die eine Zone mit biotinyliertem Antigen, eine Detektorzone und eine Abfangzone stromabwärts von der Zone mit biotinyliertem Antigen und der Detektorzone enthält, wobei die Zone mit biotinyliertem Antigen das an Biotin gebundene Antigen enthält, die Detektorzone Detektorteilchen umfaßt, die mit einem Anti-Biotin-Antikörper beschichtet sind, und die Abfangzone an die chromatographische Matrix gebunden das Antigen oder ein Antigenanalogon enthält.

2. Vorrichtung gemäß Anspruch 1, wobei die chromatographische Matrix aus der Gruppe ausgewählt ist, die aus Siliciumoxid, Nylon, Glasfaser, Polyacrylamid, Aluminiumoxid und Nitrocellulose besteht.

3. Vorrichtung gemäß Anspruch 1, wobei die Detektorteilchen eine sichtbare Ablesung ergeben.

4. Vorrichtung gemäß Anspruch 1, wobei der Detektor aus der Gruppe ausgewählt ist, die aus gefällten oder unlöslichen Metallen, gefällten oder unlöslichen Nichtmetallen, einen Farbstoff oder eine farbige Substanz enthaltenden Beuteln und gefällten oder unlöslichen Legierungen besteht.

5. Verfahren zur Bestimmung der Gegenwart eines spezifischen Antikörpers gegen ein Antigen in einer Probe, umfassend:
(i) Auftragen der Probe auf eine chromatographische Matrix einer Vorrichtung, die eine Zone mit biotinyliertem Antigen, eine Detektorzone und eine Abfangzone enthält, wobei die Probe stromaufwärts von den drei Zonen aufgetragen wird;
(ii) Wandernlassen der Probe zuerst durch die Zone mit biotinyliertem Antigen, die an Biotin gebundenes Antigen enthält, und die Detektorzone, die Detektorteilchen umfaßt, die mit einem Anti-Biotin-Antikörper beschichtet sind, so daß sich Antigen-Antikörper-Komplexe bilden, und anschließend durch die Abfangzone, die an die chromatographische Matrix gebunden das Antigen oder ein Antigenanalogon enthält, so daß die gebildeten Antigen-Antikörper-Komplexe an das Antigen oder Antigenanalogon binden; und
(iii) Beobachten der Abfangzone im Hinblick auf die Gegenwart von Teilchen, wobei die Gegenwart von Teilchen die Gegenwart des spezifischen Antikörpers anzeigt.

6. Verfahren gemäß Anspruch 5, wobei die chromatographische Matrix aus der Gruppe ausgewählt ist, die aus Siliciumoxid, Nylon, Glasfaser, Polyacrylamid, Aluminiumoxid und Nitrocellulose besteht.

7. Verfahren gemäß Anspruch 5, wobei die Detektorteilchen eine sichtbare Ablesung ergeben.

8. Verfahren gemäß Anspruch 5, wobei der Detektor aus der Gruppe ausgewählt ist, die aus gefällten oder unlöslichen Metallen, gefällten oder unlöslichen Nichtmetallen, einen Farbstoff oder eine farbige Substanz enthaltenden Beuteln und gefällten oder unlöslichen Legierungen besteht.

9. Verfahren gemäß Anspruch 5, wobei die Probe durch die Zone mit biotinyliertem Antigen wandert, bevor sie durch die Detektorzone wandert.

10. Verfahren gemäß Anspruch 5, wobei die Probe nach der Detektorzone durch die Zone mit biotinyliertem Antigen wandert.

## Revendications

1. Dispositif pour mettre en oeuvre des dosages immunologiques, en une seule étape, d'anticorps spécifiques dirigés contre un antigène, comprenant une matrice chromatographique contenant une zone d'antigène biotinylé, une zone de détection et une zone de capture en aval de ladite zone d'antigène biotinylé et de ladite zone de détection, dans lequel la zone d'antigène biotinylé contient l'antigène fixé à la biotine, ladite zone de détection comprend des matières particulaires détectrices recouvertes d'un anticorps anti-biotine, et ladite zone de capture contient l'antigène, ou un analogue d'antigène, fixé à la matrice chromatographique.

2. Dispositif selon la revendication 1, dans lequel la matrice chromatographique est choisie dans le groupe constitué par la silice, le nylon, la fibre de verre, le polyacrylamide, l'alumine, et la nitrocellulose.

3. Dispositif selon la revendication 1, dans lequel les matières particulaires détectrices produisent un affichage visible.

4. Dispositif selon la revendication 1, dans lequel le détecteur est choisi dans le groupe constitué par les métaux précipités ou insolubles, les non-métaux précipités ou insolubles, les sacs contenant un colorant ou une substance colorée et les alliages précipités ou insolubles.

5. Procédé pour déterminer la présence d'anticorps spécifique dirigé contre un antigène dans un échantillon, comprenant les étapes qui consistent à:
(i) déposer l'échantillon sur une matrice chromatographique d'un dispositif contenant une zone d'antigène biotinylé, une zone de détection et une zone de capture, ledit échantillon étant déposé en amont des trois zones;
(ii) laisser migrer ledit échantillon tout d'abord à travers ladite zone d'antigène biotinylé, qui contient de l'antigène conjugué à la biotine, et ladite zone de détection, qui contient des matières particulaires détectrices recouvertes d'un anticorps anti-biotine, de sorte qu'il se forme des complexes antigène-anticorps, et ensuite à travers ladite zone de capture, qui contient l'antigène, ou un analogue d'antigène, fixé à ladite matrice chromatographique, de sorte que les complexes antigène-anticorps formés se fixent audit antigène ou analogue d'antigène; et
(iii) observer la présence de matières particulaires dans la zone de capture, la présence de matières particulaires indiquant la présence de l'anticorps spécifique.

6. Procédé selon la revendication 5, dans lequel la matrice chromatographique est choisie dans le groupe constitué par la silice, le nylon, la fibre de verre, le polyacrylamide, l'alumine, et la nitrocellulose.

7. Procédé selon la revendication 5, dans lequel les matières particulaires détectrices produisent un affichage visible.

8. Procédé selon la revendication 5, dans lequel le détecteur est choisi dans le groupe constitué par les métaux précipités ou insolubles, les non-métaux précipités ou insolubles, les sacs contenant un colorant ou une substance colorée et les alliages précipités ou insolubles.

9. Procédé selon la revendication 5, dans lequel ledit échantillon migre à travers ladite zone d'antigène biotinylé avant de migrer à travers la zone de détection.

10. Procédé selon la revendication 5, dans lequel ledit échantillon migre à travers ladite zone d'antigène biotinylé après ladite zone de détection.
